Europäisches Patentamt

(19)  European Patent Office

Office européen des brevets

(11)  **EP 0 442 173 B1**

(12)  # EUROPEAN PATENT SPECIFICATION

(45)  Date of publication and mention
of the grant of the patent:
**23.04.1997  Bulletin 1997/17**

(51)  Int. Cl.$^6$: **C07C 269/04**

(21)  Application number: **90301482.7**

(22)  Date of filing: **13.02.1990**

(54)  **Process for the preparation of alkyl methylcarbamates**

Verfahren zur Herstellung von Alkylmethylcarbamaten

Procédé de préparation de méthylcarbamates d'alkyle

(84)  Designated Contracting States:
**BE CH DE FR GB LI NL**

(43)  Date of publication of application:
**21.08.1991  Bulletin 1991/34**

(73)  Proprietor: **Council of Scientific and
Industrial Research
New Delhi 110 001 (IN)**

(72)  Inventors:
  • **Chaudhari, Raghunath Vitthal
    Pune, Maharashtra (IN)**
  • **Gupte, Sunil Purushottam
    Pune, Maharashtra (IN)**

  • **Kelkar, Ashutosh Anant
    Pune, Maharashtra (IN)**
  • **Kolhe, Devidas Shridhar
    Pune, Maharashtra (IN)**

(74)  Representative: **Woods, Geoffrey Corlett et al
J.A. KEMP & CO.
14 South Square
Gray's Inn
London WC1R 5LX (GB)**

(56)  References cited:
**EP-A- 0 071 835          EP-A- 0 083 096
EP-A- 0 195 515**

  • **JOURNAL OF ORGANIC CHEMISTRY, vol. 49,
    no. 7, 1984, p. 1459**

## Description

The present invention relates to an improved process for the preparation of a methylcarbamate by the oxidative carbonylation of methylamine. Examples of the methylcarbamates which can be prepared by the inventive process include methyl methylcarbamate, ethyl methylcarbamate and butyl methylcarbamate.

Alkyl carbamates have in the past been manufactured by phosgination of aromatic or aliphatic amines. According to this prior art process, aromatic or aliphatic amines are reacted with phosgene to produce isocyanates which are then reacted with alcohol to produce the desired carbamates.

Unfortunately, the phosgination process employs starting materials such as phosgene and isocyanates which are toxic and therefore hazardous and this renders this particular process unsafe. Furthermore, the step of phosgination results in the generation of hydrochloric acid which is the cause of severe problems of corrosion and it will be appreciated that corrosion of production systems is particularly undesirable in such a process.

Accordingly, there has been a long-felt need to develop a process for the preparation of alkyl carbamates, particularly methyl methylcarbamate, which avoids the use of the toxic starting materials of phosgene and isocyanates. To this end there have been a number of efforts. For instance, a process for the oxidative carbonylation of aromatic amines to aryl carbamates has been reported in JP 58,146,549, 1983 and a number of catalysts for such a process selected from the elements referred to in Group VI a of the Periodic Table have been proposed (Journal of the American Chemical Society 93, 6344, 1971; Bull Chem. SAoc. Japan 57, 251, 1984 and Agew. Chem. Int. Ed. [England] 18, 692, 1979). Regrettably, such process suffer from the disadvantage that the oxidative carbonylation reactions are all stoichiometric in nature and not truly catalytic.

Another process which has been proposed for the oxidative carbonylation of amines involves the use of palladium chloride and a Lewis acid which must contain metal components capable of undergoing redox reactions such as $CuCl_2$, $FeCl_3$ and FeOCl. This process has been described in German Patents Nos. 2908250 and 2910132, U.S. Patents Nos. 4304922 and 4297560 and European Patent No. 36895. However, this further process possesses the drawback that solutions of the chlorides mentioned are highly corrosive and regeneration of the catalyst is often difficult.

More recently, there has been proposed a process for the oxidative carbonylation of aromatic amines to produce aryl carbamates in which supported noble metal catalysts are employed. Examples of the noble metals constituting such catalysts include rhodium, iridium, palladium, platinum and ruthenium. Conventionally, alkali metal halides are used as promoters in conjunction with such catalysts. Processes of this nature have been described in Japanese Patents Nos. 58 146,549, 58 144,363 and 58 150,555, all of 1983, Journal Chem. Soc. Chem. Commun., 339, 1984, Journal Org. Chem. 49, 1984 and Chemtech, 6 70, 1984.

This recent process achieves a very high yield of carbamates With almost 100% selectivity.

J. Organic Chemistry, 49, (7), 1984, pages 1458-1460, reports catalytic synthesis of carbamates by oxidative alkoxycarbonylation of amines in the presence of a platinum group metal/alkali metal halide or onium halide catalyst system. The synthesis is illustrated with reference to the preparation of ethylphenylcarbamate using aniline and ethanol as reactants.

EP-A-83096 is concerned with the production of urethane compounds by reacting at least one compound selected from the group consisting of a primary amine, a secondary amine and a urea compound with carbon monooxide and an organic hydroxyl group in the presence of molecular oxygen and/or an organic nitro compound as oxidising agent in the presence of a catalyst system comprising a platinum group metal or metal compound and a halogen-containing compound. The preferred urethane compound of ethyl N-phenylcarbamate.

It is the basic object of the present invention to provide an improved process for the preparation of alkyl N-methylcarbamates which avoids the drawbacks referred to above.

A more specific object of the invention resides in the provision of an improved process for the preparation of alkyl N-methylcarbamates which avoids the employment of hazardous or toxic compounds such as phosgene and/or methyl isocyanate as starting materials and which can be effected under mild conditions of temperature and pressure.

A further object of the invention resides in the provision of an improved process for the preparation of alkyl N-methylcarbamates by the oxidative carbonylation of methylamine.

According to the present invention, there is provided a process for the preparation of alkyl N-methylcarbamates which comprises reacting methylamine with carbon monoxide, an oxidising agent and an aliphatic monoalcohol of 1 to 10 carbon atoms or alicyclic monoalcohol of 3 to 10 carbon atoms, in the presence of a catalyst comprising (i) palladium or rhodium metal or a soluble compound thereof and (ii) an alkali metal or quaternary ammonium iodide.

The overall reactions envisaged by the process of the present invention are as follows:

$$R\,NH_2 + CO + 1/2\,O_2 \xrightarrow[R'\,OH]{Catalyst} RNHCOOR' + H_2O \qquad\qquad I$$

wherein R is methyl and R' OH is an aliphatic monoalcohol of 1 to 10 carbon atoms or an alicyclic monoalcohol of 3 to 10 carbon atoms.

The carbon monoxide employed in the process of the present invention may be pure gaseous carbon monoxide but may also contain impurities such as nitrogen and carbon dioxide. An impurity content of less than 10% volume per volume does not affect the reaction pattern and from the industrial viewpoint, it may be advantages to use carbon monoxide with small amounts of impurities. Carbon monoxide is generally employed in an amount of at least one mole per amino group of the methyl amine. A more preferred amount of carbon monoxide is from 2 to 100 moles per amino group of the methyl amine.

The oxidizing agent used in the process of the invention may be pure oxygen or a gas containing oxygen such as air. The process also tolerates the employment in some cases of an oxygen-containing gas which additionally contains other non-interfering gases such as nitrogen, argon or carbon dioxide.

The monoalcohol compound which forms one of the reactants can also function as a solvent. However, where necessary, other solvents which do not affect the reaction adversely may also be used. Exemplary of such solvents are aromatic hydrocarbons such as benzene, toluene, xylene and mesitylene, nitriles such as acetonitrile and benzonitrile, ethers such as tetrahydrofuran and 2-dioxane, ketones such as acetone and methyl ethyl ketone, amides such as N,N'-dimethyl formamide and N,N'-dimethyl acetamide, and esters such as ethyl acetate and ethyl benzoate.

Component (i) of the catalyst system which acts as a precursor can comprise palladium or rhodium metal or a soluble compound of such a metal supported, if desired, on a suitable carrier. Of these metals, palladium is specially preferred.

Specific examples of the catalyst precursors include Pd black, supported palladium catalysts such as Pd-C, Pd-$Al_2O_3$ or Pd-$CaCO_3$, and intermetallic compounds such as Pd-Se, Pd-Co or Pd-Rh. Pd black which has been prepared by various reducing agents such hydrazine hydrate, sodium formate, formaldehyde, sodium borohydride, $LiAlH_4$ and $H_2$ can also be used.

Soluble Pd compounds that can be used are $PdCl_2$, $PdBr_2$, $PdI_2$, $Pd(NO_3)_2$, $Pd(OCOCH_3)_2$, Pd-oxalate, $[Pd(NH_3)_4]X_2$, $[PdL_2X]$, $[Pd(CO)X]$ wherein X is chlorine, bromine or iodine and L is triphenyl phosphine, pyridine, isoquinoline, tributyl phosphine or benzonitrile.

Metallic rhodium, intermetallic compounds of rhodium and supported rhodium compounds similar to catalyst precursors prepared of or from palladium as described above can also be used. Soluble rhodium complexes that can be used include $RhCl_3$, $RhI_3$, $[Rh(CO)_2Cl]_2$, $RhCl(PPh_3)_3$, $RhX(CO)L_2$ wherein X is chlorine, bromine or iodine and L is triphenyl phosphine, tributyl phosphine or triphenyl arsine.

The iodide promotor which constitutes component (ii) of the catalyst system can be selected from alkali metal iodides and quarternary ammonium iodides. Preferred are sodium iodide, potassium iodide, lithium iodide, cesium iodide, tetrabutyl ammonium iodide, or tetraheptyl ammonium iodide.

The oxidative carbonylation reaction of the present invention can be carried out in a temperature range of 80°C to 350°C, more preferably between 120°C and 250°C.

Preferably, the carbonylation is carried out under a carbon monoxide partial pressure of from 5 to 6000 psig (34.5 to 41,400 kPa), more preferably between 100 to 1500 psig (690 to 10350 kPa). The partial pressure of oxygen employed is preferably between 5 and 1000 psig (34.5 to 6900 kPa), more preferably between 10 and 300 psig (69 to 2070 kPa). The ratio of CO : $O_2$ in the reactor can be in the range of from 1 : 1 to 50 : 1, preferably in the range of from 5 : 1 to 20 : 1.

In giving effect to the reaction of the present invention, it has been found convenient to employ 1 mol of catalyst per 5 to 8000 mols of methyl amine. A more preferred range comprises one mole of catalyst per 100 to 500 mols of methyl amine.

Within the catalyst, the ratio of iodide promoter to precursor is preferably in the range of from 0.1 to 10, more preferably between 0.5 and 5.

The amount of monoalcohol employed is at least one mol per mol of the methylamine. However, it is more preferable to use 3 to 100 mols of the monoalcohol per mol of the methylamine.

It has been found that the process of the present invention results in a 95% to 100% conversion of methylamine to the corresponding carbamate with from 80% to 90% selectivity for carbamate.

The carbamates produced by the process of the present invention find employment in the manufacture of isocyanates, urethane foams, coatings and fibres, pesticides and insecticides. They are particularly applicable for the production of carbamate insecticides such as carbaryl, the generic name for (1-naphthyl-N- methyl carbamate which is sold under the trade name "SEVIN", 2-sec butyl phenyl methyl carbamate popularly known as BPMC and carbofuran.

The invention will now be described in detail in the following examples.

Example 1

Methyl methyl carbamate was prepared by charging the following components to a 300 ml stirred autoclave:

3

| Methyl amine | 48.38 mmol |
|---|---|
| $PdPy_2Cl_2$ | 4.73 mmol |
| Sodium iodide | 4.72 mmol |
| Methanol | 2412.60 mmol |

The contents were heated to 150°C. Then autoclave was pressurized with 100 psig of oxygen and then further pressurized up to 1000 psig with carbon monoxide. The pressure in the reactor was maintained constant at 1000 psig, and the progress of the reaction was monitored by observing the pressure drop in the $CO/O_2$ reservoir. The reaction was continued for 6.5 hours. The reactor was then cooled and the liquid phase was analyzed by gas chromatography. GC analysis showed 86.5% conversion of methylamime and the yield of methyl methyl carbamate was 45.96 mmol.

Example 2

Methyl methyl carbamate was produced by charging the following components to a 300 ml stirred autoclave:

| Methyl amine | 64.52 mmol |
|---|---|
| $Pd (PPh_3)_2Cl_2$ | 4.72 mmol |
| Lithium iodide | 4.73 mmol |
| Methanol | 2394.6 mmol |

The reaction was carried out in accordance with the procedure described in Example 1 and the liquid phase was analyzed by gas chromatography (GC) at the end of the reaction. After 6 hours, GC analysis showed 91.2% conversion of methylamine and the yield of methyl methyl carbamate was 60.23 mmol.

Example 3

Methyl methyl carbamate was produced by charging the following components to a 300 ml stirred autoclave:

| Methyl amine | 64.52 mmol |
|---|---|
| $Pd (PhNC)_2Cl_2$ | 4.72 mmol |
| Lithium iodide | 4.72 mmol |
| Methanol | 2394.6 mmol |

The reaction was carried out in accordance with the procedure described in Example 1 and the liquid phase was analyzed by gas chromatography (GC) at the end of the reaction. After 6.5 hours, GC analysis showed 91.3% conversion of methylamine and the yield of methyl methyl carbamate was 60.12 mmol.

Example 4

Methyl methyl carbamate was produced by charging the following components to a 300 ml stirred autoclave:

| Methyl amine | 80.6 mmol |
|---|---|
| Palladium metal | 4.72 mmol |
| Lithium iodide | 4.73 mmol |
| Methanol | 2375.6 mmol |

The reaction was carried out in accordance with the procedure described in Example 1 and the liquid phase was analyzed by gas chromatography (GC) at the end of the reaction. After 5.5 hours, GC analysis showed 94.5% conversion of methylamine and the yield of methyl methyl carbamate was 70.92 mmol.

Example 5

Methyl methyl carbamate was produced by charging the following components to a 300 ml stirred autoclave:

| Methyl amine | 80.6 mmol |
|---|---|
| Palladium chloride | 4.72 mmol |
| Lithium iodide | 4.73 mmol |
| Methanol | 2375.6 mmol |

The reaction was carried out in accordance with the procedure described in Example 1 and the liquid phase was analyzed by gas chromatography (GC) at the end of the reaction. After 5.5 hours, GC analysis showed 94.1% conversion of methylamine and the yield of methyl methyl carbamate was 70.92 mmol.

Example 6

Methyl methyl carbamate was produced by charging the following components to a 300 ml stirred autoclave:

| Methyl amine | 80.6 mmol |
|---|---|
| Palladium metal | 4.72 mmol |
| Potassium iodide | 4.71 mmol |
| Methanol | 2375.6 mmol |

The reaction was carried out in accordance with the procedure described in Example 1 except that potassium iodide replaced lithium iodide and the liquid phase was analyzed by gas chromatography (GC) at the end of the reaction. After 6.2 hours, GC analysis showed 88.0% conversion of methylamine and the yield of methyl methyl carbamate was 70.9 mmol.

Example 7

Methyl methyl carbamate was produced by charging the following components to a 300 ml stirred autoclave:

| Methyl amine | 80.6 mmol |
|---|---|
| Palladium metal | 4.72 mmol |
| Tetrabutyl ammonium iodide | 4.73 mmol |
| Methanol | 2375.6 mmol |

The reaction was carried out in accordance with the procedure described in Example 1 except that tetrabutyl ammonium iodide replaced lithium iodide and the liquid phase was analyzed by gas chromatography (GC) at the end of the reaction. After 6.5 hours, GC analysis showed 86.8% conversion of methylamine and the yield of methyl methyl carbamate was 72.4 mmol.

Example 8

Methyl methyl carbamate was produced by charging the following components to a 300 ml stirred autoclave:

| Methyl amine | 80.6 mmol |
|---|---|
| Palladium metal | 4.72 mmol |
| Sodium iodide | 4.73 mmol |
| Methanol | 2375.6 mmol |

The reaction was carried out in accordance with the procedure described in Example 1 except that sodium iodide replaced lithium iodide and the liquid phase was analyzed by gas chromatography (GC) at the end of the reaction. After 6.5 hours, GC analysis showed 89.1% conversion of methylamine and the yield of methyl methyl carbamate was 74.0 mmol.

Example 9

Methyl methyl carbamate was produced by charging the following components to a 300 ml stirred autoclave:

| Methyl amine | 80.6 mmol |
|---|---|
| Palladium metal | 4.72 mmol |
| Lithium iodide | 4.73 mmol |
| Methanol | 2375.6 mmol |

The reaction was carried out in accordance with the procedure described in Example 1 except that the reaction temperature was 170°C instead of 150°C. The liquid phase was analyzed by gas chromatography (GC) at the end of the reaction. After 5.5 hours, GC analysis showed 98.0% conversion of methylamine and the yield of methyl methyl carbamate was 72.2 mmol.

Example 10

Methyl methyl carbamate was produced by charging the following components to a 300 ml stirred autoclave:

| Methyl amine | 32.25 mmol |
|---|---|
| Palladium metal | 4.72 mmol |
| Lithium iodide | 4.73 mmol |
| Methanol | 2431.2 mmol |

The reaction was carried out in accordance with the procedure described in Example 1 and the liquid phase was analyzed by gas chromatography (GC) at the end of the reaction. After 5.6 hours, GC analysis showed 98.5% conversion of methylamine and the yield of methyl methyl carbamate was 28.38 mmol.

Example 11

Methyl methyl carbamate was produced by charging the following components to a 300 ml stirred autoclave:

| Methyl amine | 80.6 mmol |
|---|---|
| $RhCl_3 . 3H_2O$ | 4.72 mmol |
| Sodium iodide | 4.73 mmol |
| Methanol | 2283.5 mmol |

The reaction was carried out in accordance with the procedure described in Example 1 and the liquid phase was analyzed by gas chromatography (GC) at the end of the reaction. After 5.2 hours, GC analysis showed 95.2% conversion of methyl amine and the yield of methyl methyl carbamate was 68.51 mmol.

The present invention presents a number of advantages. As far as the applicants are aware, their process represents the first occasion that methyl carbamates, particularly methyl methyl carbamate, are being prepared by oxidative carbonylation. The invention thus makes it possible to employ methyl amine to prepare such carbamates and this ability is the invention's main advantage.

Secondly, the inventive process is performed in a single step without the formation of toxic methyl isocyanate and is far less hazardous to perform than the conventional phosgenation process.

Finally, the process of the present invention operates under milder reaction conditions of temperature and pressure than hitherto known processes and a high selectivity to carbamates is achieved. The catalyst can be separated from the reaction crude simply by filtration and can be reused several times. Overall, therefore, the process of the present invention is expected to display considerable economical advantages over the known art.

**Claims**

1. A process for the preparation of a methylcarbamate which comprises reacting methylamine with carbon monoxide, an oxidising agent and an aliphatic monoalcohol of 1 to 10 carbon atoms or alicyclic monoalcohol of 3 to 10 carbon atoms, in the presence of a catalyst comprising (i) palladium or rhodium metal or a soluble compound thereof and (ii) an alkali metal or quaternary ammonium iodide.

2. A process as claimed in claim 1 wherein said carbon monoxide is employed in an amount of at least one mole, preferably from 2 to 100 moles, per mole of said amine.

3. A process as claimed in claim 1 or 2 wherein said reaction is effected in the presence of a solvent.

4. A process as claimed in claim 3 wherein said solvent is an aromatic hydrocarbon such as benzene, toluene, xylene or mesitylene, a nitrile such as acetonitrile or benzonitrile, an ether such as tetrahydrofuran or 2-dioxane, a ketone such as acetone or methyl ethyl ketone, an amine such as N,N'-dimethyl formamide or N,N'-dimethyl acetamide, or an ester such as ethyl acetate or ethyl benzoate.

5. A process as claimed in any of claims 1 to 4 wherein the catalyst system is supported on a suitable carrier.

6. A process as claimed in any of claims 1 to 5 wherein the said reaction is carried out at a temperature of from 80°C to 350°C, preferably from 120°C to 250°C.

7. A process as claimed in any of claims 1 to 6 wherein said reaction is effected under a partial pressure of carbon monoxide of from 5 to 6000 psig (34.5 to 41,400 kPa), preferably from 100 to 1500 psig (690 to 10350 kPa), and a partial pressure of oxygen of from 5 to 1000 psig (34.5 to 6900 kPa), preferably 10 to 300 psig (69 to 2070 kPa).

8. A process as claimed in any of claims 1 to 7 wherein the ratio of carbon monoxide to oxygen in said reaction is in the range of from 1:1 to 50:1, preferably from 5:1 to 20:1.

9. A process as claimed in any of claims 1 to 8 wherein said catalyst is employed in an amount of 1 mol of catalyst per 5 to 8000 mols of amine, preferably 1 mol of catalyst per 100 to 500 mols of amine.

10. A process as claimed in any of claims 1 to 9 wherein the ratio of component (ii) to component (i) in said catalyst is in the range of from 0.1 to 10, preferably from 0.5 to 5.

11. A process as claimed in any of claims 1 to 10 wherein the monoalcohol is employed in an amount of at least 1 mol per mol of methylamine, preferably in an amount of from 3 to 100 mols per mol of methylamine.

12. A process as claimed in any of claims 1 to 11 wherein the monoalcohol employed is methanol.

**Patentansprüche**

1. Verfahren zur Herstellung eines Methylcarbamats mit den folgenden Schritten:
Umsetzen von Methylamin mit Kohlenmonoxid, einem Oxidationsmittel und einem aliphatischen Monoalkohol m:t 1 bis 10 Kohlenstoffatomen oder einem alicyclischen Monoalkohol mit 3 bis 10 Kohlenstoffatomen in der Gegenwart eines Katalysators der (i) Palladium- oder Rhodiummetall oder eine lösliche Verbindung davon und (ii) ein Alkalimetall- oder quaternäres Ammoniumiodid enthält.

2. Verfahren gemäß Anspruch 1, wobei das Kohlenmonoxid in einer Menge von wenigstens einem Mol pro Mol des Amins eingesetzt wird, bevorzugt von 2 bis 100 Mol.

3. Verfahren gemäß Anspruch 1 oder 2, wobei die Reaktion in Gegenwart eines Lösungsmittels bewirkt wird.

4. Verfahren gemäß Anspruch 3, wobei das Lösungsmittel ein aromatischer Kohlenwasserstoff wie Benzol, Toluol, Xylol oder Mesitylen ist, ein Nitril, wie Acetonitril oder Benzonitril, ein Ether wie Tetrahydrofuran oder 2-Dioxan, ein Keton, wie Aceton oder Methylethylketon, ein Amin, wie N,N'-Dimethylformamid oder N,N'-Dimethylacetamid, oder ein Ester, wie Ethylacetat oder Ethylbenzoat.

5. Verfahren gemäß einem der Ansprüche 1 bis 4, wobei das Katalysatorsystem von einem geeigneten Träger getragen wird.

6. Verfahren gemäß einem der Ansprüche 1 bis 5, wobei die Reaktion bei einer Temperatur von 80°C bis 350°C durchgeführt wird, bevorzugt von 120°C bis 250°C.

7. Verfahren gemäß einem der Ansprüche 1 bis 6, wobei die Reaktion unter einem Kohlenmonoxidpartialdruck von 5 bis 6.000 psig (34,5 bis 41.400 kPa) durchgeführt wird, bevorzugt von 100 bis 1.500 psig (690 bis 10.350 kPa), und einem Sauerstoffpartialdruck von 5 bis 1.000 psig (34,5 bis 6.900 kPa), bevorzugt 10 bis 300 psig (69 bis 2.070 kPa).

8. Verfahren gemäß einem der Ansprüche 1 bis 7, wobei das Verhältnis von Kohlenmonoxid zu Sauerstoff bei der Reaktion in dem Bereich von 1:1 bis 50:1 liegt, bevorzugt von 5:1 bis 20:1.

9. Verfahren gemäß einem der Ansprüche 1 bis 8, wobei der Katalysator in einer Menge von 1 Mol Katalysator pro 5 bis 8.000 Mol Amin eingesetzt wird, bevorzugt 1 Mol Katalysator pro 100 bis 500 Mol Amin.

10. Verfahren gemäß einem der Ansprüche 1 bis 9, wobei das Verhältnis von Komponente (ii) zu Komponente (i) in dem Katalysator in dem Bereich von 0,1 bis 10 liegt, bevorzugt von 0,5 bis 5.

**11.** Verfahren gemäß einem der Ansprüche 1 bis 10, wobei der Monoalkohol in einer Menge von wenigstens 1 Mol pro Mol Methylamin eingesetzt wird, bevorzugt in einer Menge von 3 bis 100 Mol pro Mol Methylamin.

**12.** Verfahren gemäß einem der Ansprüche 1 bis 11, wobei der eingesetzte Monoalkohol Methanol ist.

**Revendications**

**1.** Procédé de préparation d'un méthylcarbamate qui comprend la réaction de la méthylamine avec le monoxyde de carbone, un agent oxydant et un monoalcool aliphatique de 1 à 10 atomes de carbone ou un monoalcool alicyclique de 3 à 10 atomes de carbone, en présence d'un catalyseur comprenant (i) du palladium ou du rhodium métallique ou un composé soluble de ceux-ci et (ii) un iodure alcalin ou d'ammonium quaternaire.

**2.** Procédé selon la revendication 1, dans lequel ledit monoxyde de carbone est employé en une quantité d'au moins 1 mol, de préférence de 2 à 100 mol, par mole de ladite amine.

**3.** Procédé selon la revendication 1 ou 2, dans lequel ladite réaction est conduite en présence d'un solvant.

**4.** Procédé selon la revendication 3, dans lequel ledit solvant est un hydrocarbure aromatique tel que le benzène, le toluène, le xylène ou le mésitylène, un nitrile tel que l'acétonitrile ou le benzonitrile, un éther tel que le tétrahydrofurane ou le 2-dioxane, une cétone telle que l'acétone ou la méthyléthylcétone, une amine telle que le N,N'-diméthylformamide ou le N,N'-diméthylacétamide ou un ester tel que l'acétate d'éthyle ou le benzoate d'éthyle.

**5.** Procédé selon l'une quelconque des revendications 1 à 4, dans lequel le système catalytique est supporté sur un support approprié.

**6.** Procédé selon l'une quelconque des revendications 1 à 5, dans lequel ladite réaction est conduite à une température de 80°C à 350°C, de préférence de 120°C à 250°C.

**7.** Procédé selon l'une quelconque des revendications 1 à 6, dans lequel ladite réaction est conduite sous une pression partielle de monoxyde de carbone de 5 à 6 000 psig (34,5 à 41 400 kPa), de préférence de 100 à 1 500 psig (690 à 10 350 kPa) et sous une pression partielle d'oxygène de 5 à 1 000 psig (34,5 à 6 900 kPa), de préférence de 10 à 300 psig (69 à 2 070 kPa).

**8.** Procédé selon l'une quelconque des revendications 1 à 7, dans lequel le rapport du monoxyde de carbone à l'oxygène dans ladite réaction est situé dans le domaine de 1 : 1 à 50 : 1, de préférence de 5 : 1 à 20 : 1.

**9.** Procédé selon l'une quelconque des revendications 1 à 8, dans lequel ledit catalyseur est employé en une quantité de 1 mol de catalyseur pour 5 à 8 000 mol d'amine, de préférence de 1 mol de catalyseur pour 100 à 500 mol d'amine.

**10.** Procédé selon l'une quelconque des revendications 1 à 9, dans lequel le rapport du constituant (ii) au constituant (i) dans ledit catalyseur est situé dans le domaine de 0,1 à 10, de préférence de 0,5 à 5.

**11.** Procédé selon l'une quelconque des revendications 1 à 10, dans lequel le monoalcool est employé en une quantité d'au moins 1 mol par mole de méthylamine, de préférence en une quantité de 3 à 100 mol par mole de méthylamine.

**12.** Procédé selon l'une quelconque des revendications 1 à 11, dans lequel le monoalcool employé est le méthanol.